# EUROPEAN PATENT SPECIFICATION

(11) **EP 1 853 548 B1**
(45) Date of publication and mention of the grant of the patent: **13.05.2009**
(21) Application number: 05804725.9
(22) Date of filing: 01.12.2005
(51) Int. Cl.: C07C 201/08, C07C 205/12, C07C 205/58, C07C 51/093, C07C 63/70

(54) **PROCESS FOR THE PREPARATION OF BENZOIC ACID DERIVATIVES VIA A NEW INTERMEDIATE OF SYNTHESIS**
VERFAHREN ZUR HERSTELLUNG VON BENZOESÄUREDERIVATEN ÜBER EIN NEUES SYNTHESEZWISCHENPRODUKT
PROCEDE ET METHODES DE PREPARATION DE GABAPENTINE ET DE PRODUITS INTERMEDIAIRES DU GABAPENTINE

(30) Priority: 22.02.2005 IT MI20050027
(43) Date of publication of application: 14.11.2007
(73) Proprietor: MITENI S.p.A., 20138 Milan (IT)
(72) Inventor: MONDINI, Simonetta,, I-20138 Milan (IT); DALL'AVA, Mirco,, I-20138 Milan (IT); GUERRATO, Alfredo,, I-20138 Milan (IT)
(74) Representative: Trupiano, Federica
(86) International application number: PCT/IB2005/003622
(87) International publication number: WO 2006/090210

(56) References cited:
- EP-A- 0 303 291
- EP-A- 0 583 705
- WO-A-01/83459
- WO-A-87/07602
- WO-A-93/09077
- DE-C- 82 927
- US-A- 4 045 502

## Description

### FIELD OF THE INVENTION

The subject-matter of the present invention is the preparation of 2-chloro-4-fluoro-5-nitrobenzoic acid and derivatives thereof, including the acyl chloride thereof and a novel synthetic intermediate. In particular, the subject-matter of the invention is a process for the preparation of the above-mentioned derivatives by nitration of 2-chloro-4-fluorobenzotrichloride and the transformation of the novel synthetic intermediate thus obtained into the acid and derivatives thereof, including the corresponding acyl chloride, or by a process comprising the nitration and simultaneous hydrolysis of said 2-chloro-4-fluorobenzotrichloride.

### TECHNICAL BACKGROUND

2-chloro-4-fluoro-5-nitrobenzoic acid is a known product.

Its preparation is described in the literature , solely by nitration of 2-chloro-4-fluorobenzoic acid, just as reported, for example, in WO87/07602, EP0863142 and WO01/83459.

The reaction described has considerable drawbacks, including the fact that the starting product, 2-chloro-4-fluorobenzoic acid is a solid, and hence not so easily handled compared to a liquid compound at room temperature. Furthermore, its nitration requires significant volumes of sulfuric acid and, by no means least, nitration of 2-chloro-4-fluorobenzoic acid leads to the production of considerable amounts of impurities, such as unwanted by-products.

WO87/07602 discloses, in a generic way, a nitration reaction on benzoic acid derivatives and on benzyl chloride derivatives. In example 1, the nitration of the benzoic acid derivative is disclosed, by using a 1/1 (v/v) mixture of nitric acid and sulfuric acid. However, it is not clear what product is obtained, as it shows a m.p. equal to 183-185°C, whereas we know the m.p. of the product is about 146-150°C.

WO93/09077 discloses i.a. the conversion of fluorinated benzotrichlorides into fluorinated benzoic acids.

### SUMMARY OF THE INVENTION

The aim of the present invention is to provide an alternative synthetic process for 2-chloro-4-fluoro-5-nitrobenzoic acid and derivatives thereof including the acyl chloride, by a novel and original preparation process, with respect to the nitration process described in the aforementioned references, furthermore obviating the drawbacks of said nitration.

Indeed, it has now been found that it is possible to prepare 2-chloro-4-fluoro-5-nitrobenzoic acid by nitration, not of the corresponding non-nitrated acid, but of 2-chloro-4-fluorobenzotrichloride, the reaction proceeding via 2-chloro-4-fluoro-5-nitrobenzotrichloride, a novel and versatile reaction intermediate which is easily converted into the desired acid or the acyl chloride thereof or into other possible compounds.

### DETAILED DESCRIPTION OF THE INVENTION

Hence, according to one aspect thereof, the object of the present invention relates to a process for the preparation of 2-chloro-4-fluoro-5-nitrobenzoic acid and derivatives thereof, of formula (I): 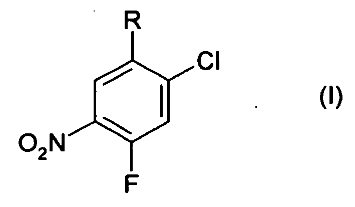 wherein
- R is a -COOH, -COOR', -CO-Hal, -CONR"R"', -COSR"' or -CN group,
- Hal represents a halogen atom,
- R' represents a linear or branched alkyl, alkenyl or alkynyl group; an optionally substituted aryl group;
- R" and R"' represent, independently, a hydrogen atom; a linear or branched alkyl, alkenyl or alkynyl group; an optionally substituted aryl or arylalkyl group;
comprising:
a) reacting 2-chloro-4-fluorobenzotrichloride of formula (II): 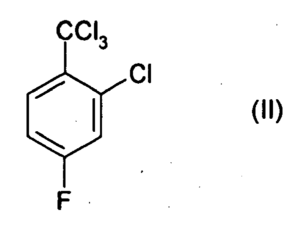 with a sulfonitric mixture to give the intermediate compound 2-chloro-4-fluoro-5-nitrobenzotrichloride of formula (III): 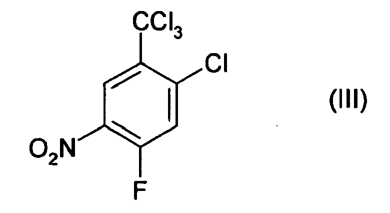
b) hydrolysing the intermediate to give the acid of formula (I) wherein R is - COOH; and optionally
c) converting the acid thus obtained into a derivative thereof.

According to the present invention, by halogen is meant a substituent selected from chlorine, bromine, fluorine and iodine, with chlorine being a particularly preferred substituent.

In particular, the preparation of the compound of formula (I) wherein R is -COCl (R = -CO-Hal with Hal = Chlorine) may proceed according to any of the following steps, including:
c') treating the acid of formula (I) wherein R is -COOH with classic chlorinating agents; or alternatively
c") reacting 2-chloro-4-fluoro-5-nitrobenzotrichloride of formula (III) with 2-chloro-4-fluoro-5-nitrobenzoic acid (formula (I) wherein R is OH) in 1/1 molar ratios,
said stages (c') and (c") providing the compound of formula (I) wherein R is -COCl.

Examples of linear or branched alkyl, alkenyl and alkynyl groups include lower alkyls such as methyl, ethyl, propyl, isopropyl, n-butyl, sec-butyl, t-butyl, iso-pentyl, etc., optionally, whenever possible, mono- or poly-unsaturated.

Examples of optionally substituted aryl and arylalkyl groups include phenyl, naphthyl, diphenyl, benzyl, etc., optionally mono or poly-substituted with linear or branched alkyl, alkenyl or alkynyl groups, with halogens or with functional groups such as carboxyls (optionally esterified), primary, secondary or tertiary amines etc..

The starting product, 2-chloro-4-fluorobenzotrichloride of formula (II), is a liquid, easily measurable product, the preparation of which is described in the literature for example in US 4,711,905 (column 17) in which it is synthesised via the photochlorination of the corresponding toluenic derivative.

According to the present invention, by "sulfonitric mixture" is meant a mixture composed of concentrated nitric acid and either concentrated sulfuric acid or oleum, wherein the nitric acid content in said sulfonitric mixture is 30% by weight with respect to the total mixture weight.

According to one advantageous aspect of the invention, the nitric acid content in the sulfonitric mixture is less than 30% by weight, for example comprised of between 15 and 30%. Indeed, it has been observed that said sulfonitric mixture is particularly advantageous when used on an industrial scale.

According to one advantageous aspect, the nitration step (a) is performed with a nitric acid/compound of formula (I) ratio greater than 1/1, even better if greater than 2/1 (mol/mol), for example with a ratio of 2.2/1 or even 3/1 or higher.

The temperature of the nitration reaction (a) is at least slightly lower than room temperature, advantageously comprised of between 0 and 20°C, for example comprised of between 10 and 20°C or between 0°C and 10°C. Temperatures less than 0°C may likewise be used but involve longer reaction times, while temperatures above room temperature do not always give the selective nitration of the desired position in the benzene ring.

In particular, it has been observed that using a temperature comprised of between 0-10°C, for example around 0-5°C, is particularly advantageous, above all when operating in the presence of a sulfonitric mixture with a nitric acid content of less than 30% by weight, allowing greater handleability of the reaction mixture with reduced production of by products.

Hydrolysis (b) of the compound of formula (III) to give the acid form, is generally performed in an acid medium, for example in the presence of hydrochloric, hydrobromic, acetic and/or sulfuric acid, at a temperature comprised of between room temperature and the reaction mixture reflux temperature, for example at a temperature greater than 50°C, advantageously comprised of between 80 and 120°C. With certain types of hydrolysis, it may be possible to add a suitable catalyst, such as for example FeCl₃. The technical details of the hydrolysis step (b) are provided in the experimental section of this description.

If desired, or indeed necessary, the acid of formula (I) (wherein R = -COOH) may be purified according to known techniques, for example by washing with water, advantageously in a water/alcohol mixture.

For the good outcome of the process of the invention according to the aforementioned steps (a), (b), (c') or (c"), it is essential that the sulfonitric mixture be used in pre-mixed form, instead of the starting product being dissolved firstly in sulfuric acid with nitric acid then being added later.

Indeed, it has been observed that when the starting product of formula (II) comes into contact firstly with sulfuric acid or oleum and then with nitric acid, then besides the desired nitration, the hydrolysis of the trichloride group to the acid form is also obtained without the formation of the 2-chloro-4-fluoro-5-nitrobenzotrichloride intermediate of formula (III), which as already mentioned, is a novel, potentially versatile compound with wide range of potential uses in the synthesis of organic compounds, that may be useful for example in the preparation of derivatives of said acid and particularly the compound of formula (I) wherein R is as defined previously but other than -COOH.

The process for the preparation of the compound of formula (I) wherein R is -COOH by the nitration and simultaneous hydrolysis of 2-chloro-4-fluorobenzotrichloride of formula (II) comprising of the addition of said compound of formula (II) in sulfuric acid or oleum, with the subsequent addition of an appropriate quantity of nitric acid to the mixture, represents however a further aspect of the present invention. The details of this synthetic process are provided in the experimental section of the present description.

In general, for carrying out the process of stage (a) of the invention, the starting product of formula (II) may be added slowly (over several hours, for example around 2-3 hours) into the pre-chilled sulfonitric mixture and kept at a temperature comprised of between 0 and 10°C, the reaction mixture may then be left at said temperature for several hours until the nitration reaction is complete. Those skilled in the art are naturally able to check the progress of the reaction by means of conventional chromatographic techniques.

Once the reaction is complete (i.e. when the starting product can no longer be detected) the reaction mixture may be treated according to known techniques for the isolation of the intermediate product of formula (III). For example, the desired product may be precipitated by treatment with ice water, and then isolated by filtration or be extracted from the suspension formed by using an appropriate solvent immiscible in water, for example a chlorinated solvent. Afterwards, using conventional isolation techniques, the compound of formula (III) may be obtained with good yield and high purity. Said compound may be used directly for subsequent transformations without any further purification.

2-Chloro-4-fluoro-5-nitrobenzotrichloride of formula (III) is a novel compound and represents a further aspect of the present invention.

The acid obtained from step (b) may be transformed into a derivative thereof (R is as defined above but # -COOH) according to techniques well known to those skilled in the art and/or described in the literature.

The transformation of the acid of formula (I) wherein R is -COOH into the acyl chloride thereof of formula (I) wherein R is -COCl (R = -CO-Hal with Hal = Chloride) may be particularly useful. Said reaction may be carried out either by chlorination of the corresponding acid according to conventional techniques, for example through the action of chlorinating agents such as thionyl chloride, under reaction conditions well known to those skilled in the art or, according to a further aspect of the present invention, by reacting 2-chloro-4-fluoro-5-nitrobenzotrichloride of formula (III) with 2-chloro-4-fluoro-5-nitrobenzoic acid.

Thus, according to a further aspect thereof, the present invention also relates to a process for the preparation of the compound of formula (I) wherein R is -COCl comprising reacting the 2-chloro-4-fluoro-5-nitrobenzotrichloride of formula (III) and 2-chloro-4-fluoro-5-nitrobenzoic acid (formula (I) wherein R is -COOH) in a molar ratio of 1/1, preferably in the presence of a Lewis acid, such as for example FeCl₃ or ZnCl₂, at a temperature comprised of between room temperature and 150-160°C, for example around 50°C or more advantageously around 80°C. The reaction is exothermic and produces hydrochloric acid. The technical details of said preparation are reported in the following experimental section.

Alternatively, it is possible to react the 2-chloro-4-fluoro-5-nitrobenzotricloride of formula (III) with water in the presence of a catalyst such as FeCl₃ or FeSO₄, or even in a weakly acidic medium, for example in the presence of dilute sulfuric acid, to obtain the of 2-chloro-4-fluoro-5-nitrobenzoic acid chloride; in this case, it is advantageous to operate at temperatures ranging between 120 and 160°C and the measured quantity of water must be added slowly and constantly in order to avoid inactivation of the catalyst and hydrolysis of the compound into its acid form.

As already mentioned, the novel compound of formula (III) is a particularly versatile chemical intermediate and may be used, for example, as a starting product for the preparation of 2-chloro-4-fluoro-5-nitrobenzotrifluoride, for example by halogen exchange reaction with hydrofluoric acid (HF).

The various aspects of the invention will now be non-limitingly illustrated by means of the following examples.

### EXPERIMENTAL SECTION

### EXAMPLE 1

### Preparation of 2-chloro-4-fluoro-5-nitrobenzotrichloride (formula (III) (according to step (a))

Into a 500 ml 4-necked round-bottomed flask, equipped with mechanical means of stirring, means of cooling, thermometer and dropping funnel, 150.2 g of 30% oleum, and 60.2 g of concentrated nitric acid (99%) are added while maintaining the temperature below 10°C. To the sulfonitric mixture thus obtained 115.6 g of 2-chloro-4-fluorobenzotrichloride are added over 2.5 hours while maintaining the temperature between 0 and 10°C. The reaction is left at said temperature for a further 2 hours to achieve completion, then poured into an ice/water mixture and the precipitation of a white solid is immediately observed. 300 g of dichloromethane is added to the mixture and the phases separated, the organic phase is washed with a solution of NaHCO₃, then dried over magnesium sulfate, filtered and evaporated to dryness. 132 g of the title product (titre by GC = 93.7%, yield 93%) are obtained as a white solid.
M.P. = 65-68°C
GC-MS identifies ion 256-262 [M-Cl]⁺
¹H NMR (spectra have been performed in CD₃COCD₃)
doublet signal at 7.91 ppm ³J_{HF} 9.91 Hz
doublet signal at 8.93 ppm ⁴J_{HF} 7.63 Hz
(reference 2.03 ppm on the central line of the residual acetone signal)
¹⁹F NMR
Singlet signal at -113.68 ppm
(trifluoroacetic acid reference, the signal of which is given at -77.00 ppm)
¹³C NMR (coupled spectrum)

| | |
|---|---|
| C(1) | 136.91 ppm ddd (⁴J_{CF} 4.7 Hz, ²J_{CH} 10.9 Hz, ³J_{CH} 6.0 Hz) |
| C(2) | 141.01 ppm ddd (³J_{CF} 11.0 Hz, ²J_{CH} 11.1 Hz, ³J_{CH} 5.0 Hz) |
| C(3) | 125.16 ppm dd (¹J_{CH} 175.5 Hz, Hz, ³J_{CF} 25.1 Hz) |
| C(4) | 157.26 ppm ddd (¹J_{CF} 271.9 Hz, ²J_{CH} 9.36 Hz, ³J_{CH} 5.9 Hz) |
| C(5) | 136.07 ppm m |
| C(6) | 127.09 ppm dd (¹J_{CH} 171.4 Hz, ³J_{CF} 1.6 Hz) |
| C(CCl₃) | 94.26 ppm dd (³J_{CH} 6.4 Hz, ⁵J_{CF} 1.5 Hz) |

### EXAMPLE 2

### Preparation of 2-chloro-4-fluoro-5-nitrobenzotrichloride (formula (III) (according to step (a))

Into a 500 ml 4-necked, cryostatically cooled round-bottomed flask equipped with mechanical means of stirring, a thermometer, dropping funnel and a acidic gas traps, 602.8 g of sulfonitric mixture containing 22% nitric acid are loaded, and the temperature cryostatically cooled to 0°C. 251.8 g of 2-chloro-4-fluorobenzotrichloride are added by means of the dropping funnel while maintaining the temperature between 0 and 2°C. The addition is completed over 4.5 hours, and the well-stirred, homogeneously appearing mixture has a pale yellow colour. The reaction mixture is left, still at 0-4°C, for a further 2 hours for the reaction to go to completion. The reaction is monitored by GC, following the disappearance of the reagent, and the acids are then diluted with 150.4 g of deionised water. The process is performed using external cooling in order to maintain the temperature below 10°C. 20 minutes after the addition of the water to dilute the acids, still with cooling, the reaction mixture is filtered under reduced pressure to give 651.6 g of filtered acids and 328.5 g of a damp, pale yellow, coarse grained solid which is washed by stirring with deionised water for one hour. The desired product is filtered to give 304.1 g of a pale yellow coloured product upon drying.
Nitration molar yield: 96.2%.
Melting point (°C): 59-62

### EXAMPLE 3

### Preparation of 2-chloro-4-fluoro-5-nitrobenzoic acid (according, to step (b))

Into a 250 ml reaction vessel 41.6 g of 96% H₂SO₄ are loaded and 8.4 g of water added dropwise (to adjust the acid concentration to 80%). The temperature of the acid is adjusted to 70°C and the portionwise addition of 10 g of purified 2-chloro-4-fluoro-5-nitrobenzotrichloride (titre by GC = 99.5%) commenced. The temperature is then set to 100-110°C, observing the progressive formation of a white solid product dispersed in the mixture. The temperature is kept for 3 hours, then the reaction mixture cooled to 50°C and diluted with 100 g of water, and the product then extracted with 40 g of ethyl acetate at room temperature. The phases are separated and the organic solution washed with 46 g of water. Finally, the solvent is evaporated to give 7 g of the title product, as a white solid.
Titre by HPLC = 94.5%, molar yield 88%.
Melting point (°C): 148-151.

### EXAMPLE 4

### Preparation of 2-chloro-4-fluoro-5-nitrobenzoic acid (according to step (b))

Into a 250 ml reaction vessel inside a 1 I glass equipped with mechanical means of stirring, thermometer, bubble condenser, thermostatically controlled oil heater, acid vapour water traps, 750 g of 96% sulfuric acid are loaded and 75 g of deionised water added dropwise to dilute the acid to 85%. The operation is completed over 1.5 hours, reaching a maximum temperature of 45°C. The acid is then heated to 80-90°C, and the portionwise addition of 302.6 g of crude 2-chloro-4-fluoro-5-nitrobenzotrichloride commenced. The production of HCl may be observed immediately, both in the reaction mixture (seen by foaming), and in the water absorption traps. The reaction is moderately exothermic, however, the portionwise addition continues in safety, avoiding the accumulation of gas with vigorous stirring.

The temperature is kept between 80-100°C for the entire duration of the reaction, and for ¾ of the duration, the reaction has the appearance of a solution, then the acid product begins to form a suspension despite the high temperature. Addition of the organic component is completed in 3 hours 45 minutes, divided into 15 minute periods with the addition of 20-30 g each. The reaction mixture is kept stirring at 100°C for a further 3 hours, until the reaction is complete. The gas absorbed in the traps (97.5 g) is an indication of the state of completion of the reaction. The reaction mixture is then cooled to 20°C, and then the product filtered to give 233 g of a creamy white, damp solid. It is then further purified by washing with water, filtration and drying to obtain 171.0 g of product.
Hydrolysis molar yield: 82%.
Melting point (°C): 148-152.

### EXAMPLE 5

### Preparation of 2-chloro-4-fluoro-5-nitrobenzoic acid (according to step (b))

Into a 250 ml reaction vessel 50 g of 96% sulfuric acid are loaded then diluted to 80% by the addition of 10 g of water. The mixture is then heated to 70°C and then the portionwise addition of 12 g of crude nitration product 2-chloro-4-fluoro-5-nitrobenzotrichloride (titre by GC = 98%) commenced. The reaction mixture is heated to 100-110°C and kept at said temperature for 3 hours to promote the formation of the hydrolysis product. While cooling, it is diluted with the addition of 50 g of water and 50 g of ice, and the product extracted with 60 g of ethyl acetate. Following separation of the phases, the organic phase is washed with 60 g of water, and the ethyl acetate concentrated under reduced pressure. 10 g of heptane are then added and 8 g of title product isolated as a white solid.
Titre by HPLC = 96.5%, molar yield 91%.
Melting point (°C): 150-153.

### EXAMPLE 6

### Preparation 2-chloro-4-fluoro-5-nitrobenzoic acid chloride (according to step (c"))

Into a 100 ml reaction vessel 30 g of 2-chloro-4-fluoro-5-nitrobenzotrichloride and 0.85 g of FeCl3 are loaded. The mixture is heated to 50°C and 22.4 g of 2-chloro-4-fluoro-5-nitrobenzoic acid added. The temperature increases to 85°C with strong release of gaseous hydrochloric acid. The mixture becomes completely fluid and the reaction is left for 1.5 hours to proceed to completion. The disappearance of the starting product and the corresponding appearance of the title compound are monitored by gas chromatography (GC).
Final GC analysis: 94.7% 2-chloro-4-fluoro-5-nitrobenzoic acid chloride (via the methyl ester).
Melting point (°C): 53-57.

## Claims

1. A process for the preparation of 2-chloro-4-fluoro-5-nitrobenzoic acid and/or the chloride derivative thereof of formula (I): 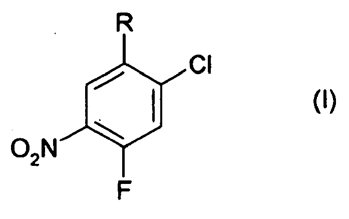 wherein
- R is a -COOH, -COOR', -CO-Hal, -CONR"R"', -COSR'" or -CN group,
- Hal represents a halogen atom,
- R' represents a linear or branched alkyl, alkenyl or alkynyl group; an optionally substituted aryl group;
- R" and R"' represent, independently, a hydrogen atom; a linear or branched alkyl, alkenyl or alkynyl group; an optionally substituted aryl or arylalkyl group,
**characterised in that**:
a) reacting 2-chloro-4-fluorobenzotrichloride of formula (II): 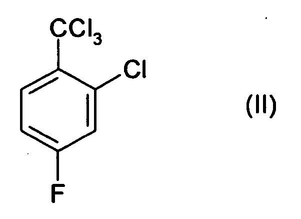 with a sulfonitric mixture to give the intermediate compound 2-chloro-4-fluoro-5-nitrobenzotrichloride of formula (III): 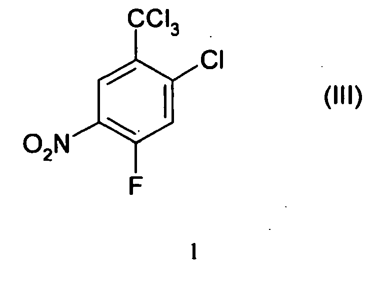
b) hydrolysing the intermediate to give the acid of formula (I) wherein R is - COOH; and optionally
c) the acid thus obtained is transformed into a derivative thereof.

2. The process according to claim 1 **characterised in that** said nitration mixture is a mixture composed of concentrated nitric acid and concentrated sulfuric acid or oleum and wherein the nitric acid content in said nitration mixture is 30% by weight with respect to the total mixture weight.

3. The process according to claims 1 or 2 **characterised in that** the nitric acid content in the nitration mixture is less than 30% by weight.

4. The process according to claims 1 to 3 **characterised in that** the nitration step (a) is carried out with a nitric acid/compound of formula (I) ratio of no less than 2/1 (mol/mol).

5. The process according to claims 1 to 4 **characterised in that** the temperature of the nitration reaction (a) is between 0 and 20°C.

6. The process according to claims 5 **characterised in that** the temperature of the nitration reaction (a) is between 0 and 10°C.

7. The process according to claims 1 to 6 **characterised in that** the hydrolysis (b) is performed in an acidic medium.

8. The process according to claims 1 to 7 for the preparation of the product of formula (I) wherein R is -COCl, **characterised in that** any of the following steps are performed:
c') treating the acid of formula (I) wherein R is -COOH with classic chlorinating agents; or alternatively
c") reacting 2-chloro-4-fluoro-5-nitrobenzotrichloride of formula (III) with 2-chloro-4-fluoro-5-nitrobenzoic acid (formula (I) wherein R is -COOH) in 1/1 molar ratios.

9. The process according to claim 8 **characterised in that** thionyl chloride is used in step (c').

10. The process according to claim 8 **characterised in that** step (c") is performed in the presence of a Lewis acid.

## Patentansprüche

1. Ein Verfahren zur Herstellung von 2-Chlor-4-fluor-5-nitrobenzoesäure und/oder dessen Chloridderivat gemäß der Formel (I): 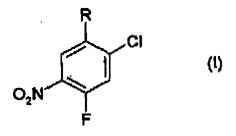 wobei
- R eine -COOH, -COOR', -CO-Hal, -CONR"R"', -COSR"' or-CN Gruppe ist,
- Hal ein Halogenatom darstellt,
- R' eine lineare oder verzweigte Alkyl-, Alkenyl- oder Alkinylgruppe; eine optional substituierte Arylgruppe darstellt;
- R" und R"' unabhängig von einander ein Wasserstoffatom; eine lineare oder verzweigte Alkyl-, Alkenyl- oder Alkinylgruppe, eine optional substituierte Aryl- oder Arylalkylgruppe darstellt,
**gekennzeichnet durch**:
a) Umsetzen von 2-Chlor-4-fluorbenzyltrichlorid der Formel (II): 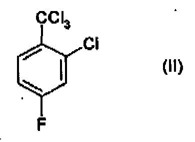 mit einer sulfosalpetrigen Mischung, um die intermediäre Verbindung 2-Chlor-4-fluor-5-nitrobenzyltrichlorid der Formel (III) zu erhalten: 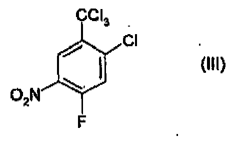
b) Hydrolyse des Intermediats, um die Säure der Formel (I) zu erhalten, worin R -COOH darstellt; und optional
c) die so erhaltene Säure in ein davon abgeleitetes Derivat umgewandelt wird.

2. Das Verfahren gemäß Anspruch 1, **dadurch gekennzeichnet, dass** die Nitriermischung aus konzentrierter Salpetersäure und konzentrierter Schwefelsäure oder Oleum besteht und der Gehalt der Salpetersäure in dieser Nitriermischung 30 Gew.-%, bezogen auf das Gewicht der gesamten Mischung beträgt.

3. Das Verfahren gemäß Anspruch 1 oder 2, **dadurch gekennzeichnet, dass** der Gehalt an Salpetersäure in der Nitriermischung geringer als 30 Gew.-% ist.

4. Das Verfahren gemäß Anspruch 1 bis 3, **dadurch gekennzeichnet, dass** der Nitrierungsschritt (a) durchgeführt wird mit einem Verhältnis von Salpetersäure zu Verbindung der Formel (I), welches nicht geringer als 2/1 (mol/mol) ist.

5. Das Verfahren gemäß Anspruch 1 bis 4, **dadurch gekennzeichnet, dass** die Temperatur der Nitrierungsreaktion (a) zwischen 0 °C und 20 °C liegt.

6. Das Verfahren gemäß Anspruch 5, **dadurch gekennzeichnet, dass** die Temperatur der Nitrierungsreaktion (a) zwischen 0 °C und 10°C liegt.

7. Das Verfahren gemäß Anspruch 1 bis 6, **dadurch gekennzeichnet, dass** die Hydrolyse (b) in saurem Medium durchgeführt wird.

8. Das Verfahren gemäß Anspruch 1 bis 7 zur Herstellung eines Produktes der Formel (I), worin R -COCI darstellt, **dadurch gekennzeichnet, dass** irgendeiner der folgenden Schritte ausgeführt wird:
c') Umsetzung der Säure der Formel (I), worin R -COOH ist, mit klassischen Chlorierungsreagenzien; oder alternativ
c") Umsetzung von 2-Chlor-4-fluor-5-nitrobenzyltrichlorid der Formel (III) mit 2-Chlor-4-fluor-5-nitrobenzoesäure (Formel (I), worin R -COOH darstellt) in molaren Verhältnissen von 1/1.

9. Das Verfahren gemäß Anspruch 8, **dadurch gekennzeichnet, dass** Thionylchlorid in Schritt (c') verwendet wird.

10. Das Verfahren gemäß Anspruch 8, **dadurch gekennzeichnet, dass** Schritt (c") in Gegenwart einer Lewis-Säure ausgeführt wird.

## Revendications

1. Procédé pour la préparation d'acide 2-chloro-4-fluoro-5-nitrobenzoique et/ou de son dérivé de chlorure de formule (I) : 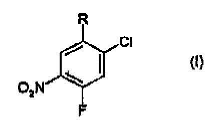 dans laquelle
- R est un groupe -COOH, -COOR', -CO-Hal, -CONR"R"', -COSR"' ou -CN,
- Hal représente un atome d'halogène
- R' représente un groupe alkyle, alcényle ou alcynyle linéaire ou ramifié ; un groupe aryle éventuellement substitué ;
- R" et R"' représentent, indépendamment, un atome d'hydrogène, un groupe alkyle, alcényle ou alcynyle linéaire ou ramifié, un groupe aryle ou arylalkyle éventuellement substitué,
**caractérisé en ce qu'**il comprend les étapes suivantes :
a) mise en réaction du 2-chloro-4-fluoro-benzotrichlorure de formule (II) : 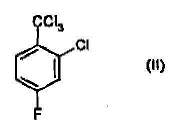 avec un mélange sulfonitrique pour donner le composé intermédiaire 2-chloro-4-fluoro-5-nitrobenzotrichlorure de formule (III) : 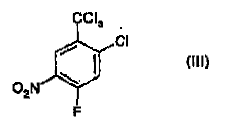
b) hydrolysation de l'intermédiaire pour donner l'acide de formule (I), dans lequel R est un -COOH et éventuellement
c) l'acide ainsi obtenu est transformé en un dérivé de celui-ci.

2. Procédé selon la revendication 1, **caractérisé en ce que** ledit mélange de nitration est un mélange composé d'acide nitrique concentré et d'acide sulfurique concentré ou d'oléum et dans lequel la teneur en acide nitrique dans ledit mélange de nitration est de 30 % en poids relativement au poids du mélange total.

3. Procédé selon les revendications 1 ou 2, **caractérisé en ce que** la teneur en acide nitrique dans le mélange de nitration est inférieure à 30 % en poids.

4. Procédé selon les revendications 1 à 3, **caractérisé en ce que** l'étape de nitration (a) est réalisée avec un rapport acide nitrique/composé de formule (I) d'au moins 2/1 (mol/mol).

5. Procédé selon les revendications 1 à 4, **caractérisé en ce que** la température de la réaction de nitration (a) est comprise entre 0 et 20 °C.

6. Procédé selon la revendication 5, **caractérisé en ce que** la température de la réaction de nitration (a) est comprise entre 0 et 10 °C.

7. Procédé selon les revendications 1 à 6, **caractérisé en ce que** l'hydrolyse (b) est réalisée en milieu acide.

8. Procédé selon les revendications 1 à 7, pour la préparation du produit de formule (I) dans laquelle R est un -COCl, **caractérisé en ce** n'importe laquelle des étapes suivantes est réalisée :
c') traitement de l'acide de formule (I) dans laquelle R est un -COOH avec des agents chlorants classiques ; ou en variante
c") réaction de 2-chloro-4-fluoro-5-nitrobenzotrichlorure de formule (III) avec de l'acide 2-chloro-4-fluoro-5-nitrobenzoique (formule (I) dans laquelle R est un -COOH) selon des rapports molaires 1/1.

9. Procédé selon la revendication 8, **caractérisé en ce que** du chlorure de thionyle est utilisé dans l'étape (c').

10. Procédé selon la revendication 8, **caractérisé en ce que** l'étape (c") est réalisée en présence d'un acide de Lewis.
